# EUROPEAN PATENT SPECIFICATION

(11) **EP 4 551 121 B1**
(45) Date of publication and mention of the grant of the patent: **08.04.2026**
(21) Application number: 23737965.6
(22) Date of filing: 30.06.2023
(51) Int. Cl.: A61B 8/06, A61B 8/08

(54) **SYSTEMS FOR ULTRASOUND IMAGE-BASED USER GUIDANCE AND FEEDBACK DURING CARDIAC VEGETATION ASPIRATION**
SYSTEME ZUR ULTRASCHALLBILDBASIERTEN BENUTZERFÜHRUNG UND RÜCKMELDUNG WÄHREND DER KARDIALEN VEGETATIONSASPIRATION
SYSTÈMES DE GUIDAGE ET DE RÉTROACTION D'UTILISATEUR BASÉS SUR DES IMAGES ÉCHOGRAPHIQUES PENDANT UNE ASPIRATION DE VÉGÉTATION CARDIAQUE

(30) Priority: 08.07.2022 US 202263359224 P
(43) Date of publication of application: 14.05.2025
(73) Proprietor: Koninklijke Philips N.V., 5656 AG Eindhoven (NL)
(72) Inventor: PAI RAIKAR, Vipul Shrihari, 5656 AG Eindhoven (NL); LI, Sibo, 5656 AG Eindhoven (NL); ANDERSON, Michael, 5656 AG Eindhoven (NL)
(74) Representative: Philips Intellectual Property & Standards
(86) International application number: PCT/EP2023/067992
(87) International publication number: WO 2024/008574

(56) References cited:
- US-A- 5 836 882
- US-A1- 2020 289 095
- ENEZATE TARIQ ET AL: "AngioVac for Minimally Invasive Removal of Intravascular and Intracardiac Masses: a Systematic Review", CURRENT CARDIOLOGY REPORTS, CURRENT SCIENCE, PHILADELPHIA, PA, US, vol. 24, no. 4, 7 February 2022 (2022-02-07), pages 377 - 382, XP037792390, ISSN: 1523-3782, [retrieved on 20220207], DOI: 10.1007/S11886-022-01658-9

## Description

### FIELD

The following relates generally to the catheter arts, vascular therapy, vegetation treatment arts, and related arts.

### BACKGROUND

Trans-venous lead extraction (TLE) procedures are required for patients that have long dwelling pacing leads implanted into the cardiac tissue to treat heart rhythm pathologies. Often, overtime, such leads require removal due to various clinical scenarios such as infections, damage or failure of lead, replacement with a new system etc. In such scenarios that warrant removal of existing leads, the physicians utilize either traction-based extraction tools or more advanced cutting and laser ablation based systems.

While many such extractions are clinically and technically successful procedures, the complication rate increases greatly with the presence of lead-tissue adhesions. These develop over time due to the natural inflammatory pathway that the body initiates when it encounters a foreign body. To complicate matters, such adhesions can develop quite a bit of mass and can manifest as wispy, stringy tissue vegetations. These can pose a significant risk of detachment during the lead extraction and can potentially lead to embolic events, particularly in the right side of the heart.

With the advent of improved real-time imaging in a cathlab, clinicians are increasingly making off-label use of thrombectomy aspiration systems to 'fish' out and aspirate these vegetations (see, e.g., B. Haupt, F. Merkle, T. Dreizler, V. Falk, and C. Starck, "Technical implementation of percutaneous thrombus aspiration using the AngioVac system," Perfusion, vol. 36, no. 4, pp. 352-356, May 2021, doi: 10.1177/0267659120946734; T. D. Richardson, R. M. Lugo, G. H. Crossley, and C. R. Ellis, "Use of a clot aspiration system during transvenous lead extraction," J. Cardiovasc. Electrophysiol., vol. 31, no. 3, pp. 718-722, 2020, doi: 10.1111/jce.14363; C. T. Starck et al., "Trans catheter aspiration of large pacemaker and implantable cardioverter-defibrillator lead vegetations facilitating safe transvenous lead extraction," EP Eur., p. euz283, Oct. 2019, doi: 10.1093/europace/euz283).

The clinical goal is to reduce the mass as much as possible prior to intervening with traditional lead extraction tools. In the hands of experienced operators, this technique has proven effective and has the potential to significantly reduce the risk involved in such procedures. However, a major concern for safely performing such a procedure is effectively managing blood loss during aspiration, particularly for hard to capture lesions.

Moreover, extracting vegetative masses using aspiration system from the heart poses a potential risk of drawing too much blood which can lead to hemodynamic instability and potential need for transfusion of multiple units of blood. The amount of blood extracted during the vegetation extraction is typically monitored by observing the collection apparatus connected at the distal end of the aspiration system. This requires manually observing the collection bag or requires a dedicated measurement apparatus. This is done either by the clinician or by operator assisting the clinician. US5836882-A discloses a method and apparatus for localizing a tip of a probe inside a biotic structure. The apparatus includes a probe with an ultrasonic transmitter attached proximate to a tip of an insertion end of the probe. The apparatus further includes a color Doppler ultrasonic imaging system coupled to a sonifying transducer and a speaker coupled to the ultrasonic imaging system which reproduces sound information indicative of the position of the probe relative to the sonifying transducer. Under an embodiment, the velocimeter serves to measure and display blood flow on a display during retrograde arterial catheterization.

The following discloses certain improvements to overcome these problems and others.

### SUMMARY OF THE DISCLOSURE.

The invention is set out in the appended set of claims. In some embodiments disclosed herein, a device for providing guidance during a procedure to remove tissue vegetation from an implant site using an aspiration catheter in which aspiration is controlled by vacuum suction includes a Doppler ultrasound imaging device configured to acquire ultrasound data of the implant site including Doppler ultrasound data of the implant site and imaging data of the implant site. An electronic processor is programmed to identify a portion of the aspiration catheter in the imaging data of the implant site; measure fluid flow through the aspiration catheter as a function of time based on the Doppler ultrasound data corresponding to the identified portion of the aspiration catheter; and provide guidance for the aspiration based on the measured fluid flow through the aspiration catheter.

In some embodiments disclosed herein, an intravascular aspiration device includes an aspiration catheter configured to aspirate material to be removed from a treatment site in at least one blood vessel of a patient using vacuum suction on the aspiration catheter. A Doppler ultrasound imaging device is configured to acquire ultrasound data of the treatment site including Doppler ultrasound data of the treatment site and imaging data of the treatment site. An electronic processor is programmed to provide real-time guidance for the aspiration of the treatment site by operations including identifying a portion of the aspiration catheter in the imaging data of the treatment site; measuring fluid flow through the aspiration catheter as a function of time based on the Doppler ultrasound data corresponding to the identified portion of the aspiration catheter; and providing guidance for the aspiration based on the measured fluid flow through the aspiration catheter.

In some embodiments disclosed herein, an intravascular aspiration method for removing tissue vegetation from an implant site in at least one blood vessel of a patient includes performing an aspiration procedure using an aspiration catheter to aspirate tissue vegetation from the implant site; acquiring ultrasound data of the implant site during the aspiration procedure including Doppler ultrasound data of the implant site and imaging data of the implant site; using an electronic processor, identifying a portion of the aspiration catheter in the imaging data of the implant site; using the electronic processor, measuring fluid flow through the aspiration catheter as a function of time based on the Doppler ultrasound data corresponding to the identified portion of the aspiration catheter; and providing guidance for the aspiration procedure based on the measured fluid flow through the aspiration catheter.

One advantage resides in helping physicians manage blood loss during vegetation aspiration procedures.

Another advantage resides in removing vegetation from leads implanted in a patient.

Another advantage resides in utilizing image-based feedback to help physicians manage blood loss during vegetation aspiration procedures.

Another advantage resides in controlling suction during vegetation aspiration procedures using image-based feedback.

A given embodiment may provide none, one, two, more, or all of the foregoing advantages, and/or may provide other advantages as will become apparent to one of ordinary skill in the art upon reading and understanding the present disclosure.

### BRIEF DESCRIPTION OF THE DRAWINGS

The disclosure may take form in various components and arrangements of components, and in various steps and arrangements of steps. The drawings are only for purposes of illustrating the preferred embodiments and are not to be construed as limiting the disclosure.
FIGURE 1 diagrammatically illustrates an aspiration device in accordance with the present disclosure.
FIGURE 2 shows an imaging device of the aspiration device of FIGURE 1.

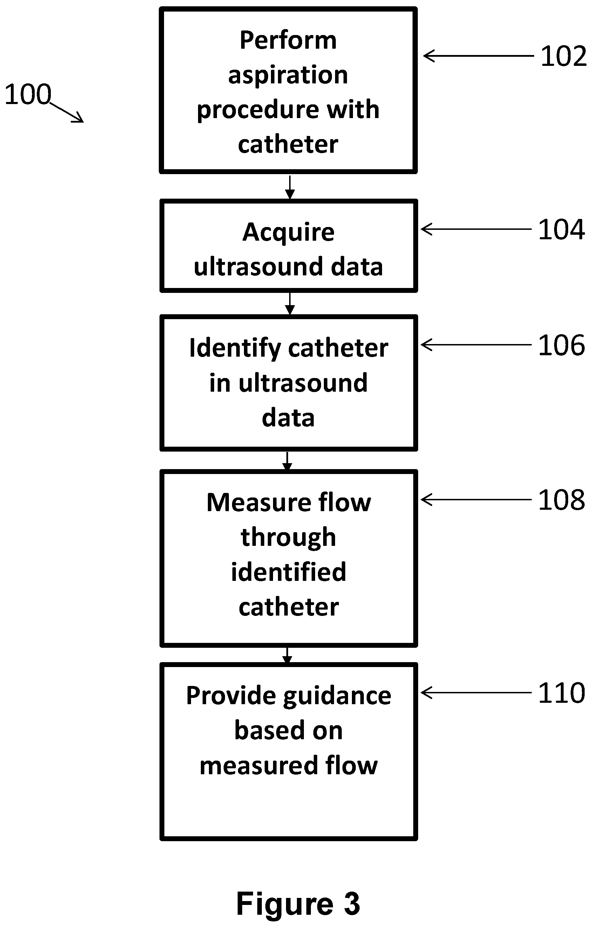

FIGURE 3 diagrammatically illustrates a method of performing an aspiration method using the device of FIGURE 1.

### DETAILED DESCRIPTION

The following relates to use of a thrombectomy aspiration system for removal of vegetation around a cardiac pacemaker lead prior to removal of the lead. The context of this is the following. Pacemaker implantation involves multiples steps. The pacemaker device (or "can") containing the electronics is implanted in the chest of the patient. Leads are inserted via an intravascular procedure so that each lead passes through the subclavian vein down through the superior vena cava, the right atrium, and its end is anchored into the right ventricle. Finally, the other end of the lead is connected to the pacemaker can. After several years, however, the attachment of the lead to the inner lumen of the venous blood vessel can become a source of clotting, thrombus, and/or infectious material, and when this happens treatment entails removal of the lead. While the lead removal is done using a dedicated intravascular tool, a potential clinical complication can occur if material from the clot, thrombus, et cetera becomes dislodged during the procedure and flows into the lungs to form an embolism.

To reduce likelihood of such an adverse clinical complication, sometimes a "pre cleaning" of the lead anchoring site is performed prior to the actual lead removal. In particular, the material built up around the anchoring site often includes a portion of wispy, stringy tissue known as vegetation, and this vegetation is of particular concern for dislodgement and a consequent embolic event. Hence, the "pre-cleaning" repurposes a conventional thrombectomy aspiration system to be used to suction away a portion of the vegetation. As a specific example, if pre procedure imaging indicates a 5 cm buildup of vegetation, it may be desirable that this be reduced to 2 cm of vegetation (or as defined by the interventionalist) by way of aspiration before initiating the lead removal procedure.

Repurposing the thrombectomy aspiration system for vegetation removal in this context introduces a concern of its own, however. Specifically, aspirations within the venous system and the right atrium, where the vegetations are the most challenging, can lead to excessive blood loss. While this can be monitored at the collection end, there is a few seconds delay between material (e.g. blood) flowing into the aspiration catheter and that material exiting at the collection end.

The following discloses repurposing a blood flow volume measurement technique disclosed in U.S. Pat. No. 6,780,155 to Li. Li discloses using Doppler ultrasound imaging to measure volumetric blood flow through a blood vessel. In the following, this is repurposed for measuring volumetric blood flow through the aspiration catheter, in combination with B mode imaging to observe the catheter itself. This technique leverages the precisely known diameter and material of the aspiration catheter to identify it quickly and accurately in the B-mode image so that the volumetric flow through the catheter can be monitored during the vegetation aspiration procedure. The approach advantageously uses existing transthoracic, transesophageal, or intracardiac ultrasound imaging that is already used to provide imaging guidance during insertion of the aspiration catheter to additionally monitor the volumetric blood flow through that catheter.

The following discloses various use cases. In one use case, if the volumetric blood flow determined by the Doppler ultrasound exceeds a maximum permissible threshold, then an audible and/or visual alarm is issued. In a variant embodiment, control feedback may automatically shut off or modulate the aspiration in this case.

In another use case, the rapid decrease in volumetric flow rate as the tip of the aspiration catheter engages the vegetation is used to provide an indication of when this occurs. In a variant, this flow rate change signal can be used to provide feedback control, for example aspirating at a lower flow rate as the tip approaches the vegetation and then ramping up the flow rate when an ultrasound-detected flow rate drop indicates engagement with vegetation.

In another use case, as the vegetation is denser than blood, the monitored volumetric flow can be used to differentiate vegetation from blood and optionally to quantitatively assess the volume of vegetation removed.

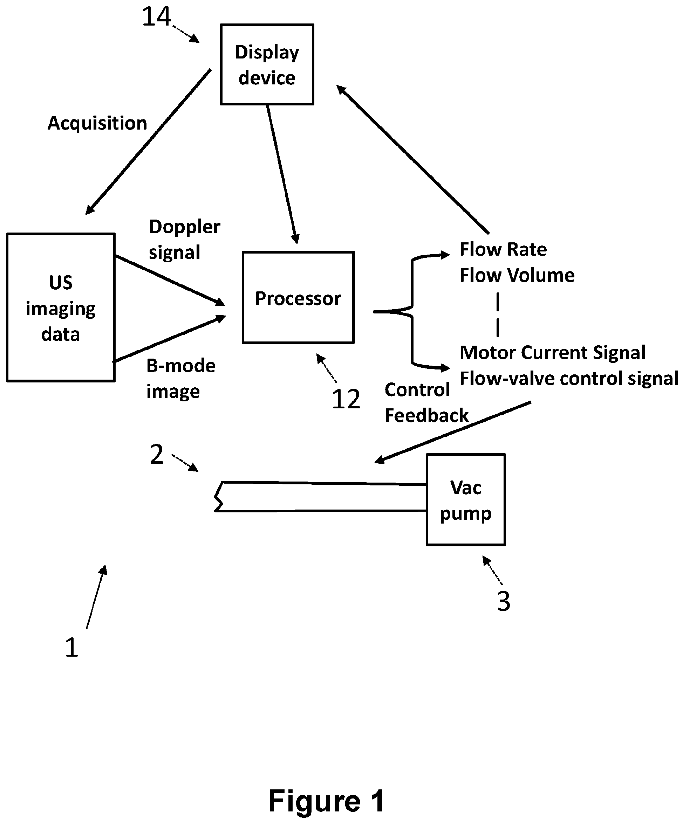

With reference to FIGURE 1, an illustrative aspiration device or apparatus **1** for providing guidance during a procedure to remove tissue vegetation from an implant site is diagrammatically shown. As shown in FIGURE 1, the apparatus **1** includes an aspiration catheter **2** configured for insertion into one or more associated blood vessels and configured to aspirate material to be removed from a treatment site in a blood vessel of a patient using vacuum suction on the aspiration catheter **2.** A vacuum source or pump **3** is operatively connected with the aspiration catheter **2** to aspirate material from the blood vessel or cardiac chamber via the aspiration catheter **2** by supplying aspiration or suction in the blood vessel or cardiac chamber.

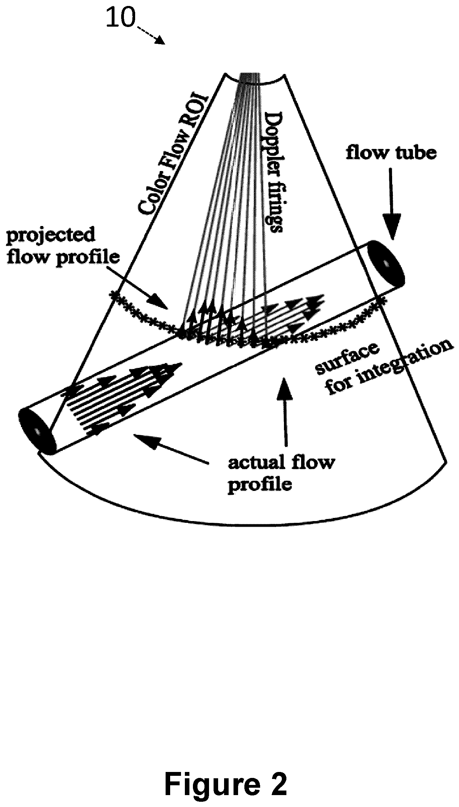

The device **1** includes a Doppler ultrasound imaging device **10** configured to acquire ultrasound data of the implant site, including Doppler ultrasound data of the implant site and imaging data of the implant site. The imaging data can comprise, for example, B-mode ultrasound imaging data. The Doppler ultrasound imaging device **10** can comprise a two-dimensional (2D) or three-dimensional (3D) color Doppler ultrasound imaging device, and perform cardiac imaging such as trans-thoracic, trans-esophageal, and intra-cardiac imaging. FIGURE 2 shows an embodiment, of the Doppler ultrasound imaging device **10** acquiring ultrasound imaging data.

An electronic processor **12** is configured to analyze the imaging data and provide guidance for a medical professional during a vegetation removal procedure. A display device **14** can display the acquired imaging data.

Referring to FIGURE 2, and with continuing reference to FIGURE 1, an illustrative embodiment of a method **100** for removing tissue vegetation from an implant site in a blood vessel of a patient using the aspiration device **1** is diagrammatically shown as a flowchart. At an operation **102,** the aspiration catheter **2** is inserted into the blood vessel, and an aspiration procedure using the aspiration catheter **2** is performed to aspirate tissue vegetation from the implant site. In some embodiments, the implant site in the blood vessel is a trans-venous lead anchor site (i.e., adhesion to the vessel wall or a site of a screw or other anchoring device for the lead), and the performing of the aspiration procedure includes using the aspiration catheter **2** to aspirate tissue vegetation from the trans-venous lead anchor site. At an operation **104,** the Doppler ultrasound imaging device **10** is operated to acquire the ultrasound data of the implant site during the aspiration procedure including the implant site and imaging data of the implant site.

At an operation **106,** the electronic processor **12** is programmed to identify a portion of the aspiration catheter **2** in the imaging data (i.e., the B-mode imaging data) of the implant site. In some examples, the identifying of the portion of the aspiration catheter **2** in the imaging data of is based in part on an *a priori* known diameter of the portion of the aspiration catheter **2.**

At an operation **108,** the electronic processor **12** is programmed to measure fluid flow (e.g., blood, vegetation, and so forth) through the aspiration catheter **2** as a function of time based on the Doppler ultrasound data corresponding to the identified portion of the aspiration catheter **2.** In some embodiments, the electronic processor **12** also measures a density of fluid forming the measured fluid flow based on the Doppler ultrasound data, and determines an amount of the material to be removed that passes through the aspiration catheter **2** based on the measured fluid flow and the measured density. In another embodiment, the electronic processor **12** is programmed to quantify an amount of the tissue vegetation at the trans-venous lead anchoring site based on the imaging data.

At an operation **110,** the electronic processor **12** is programmed to provide guidance for the aspiration based on the measured fluid flow through the aspiration catheter **2.** This can be performed in a variety of manners. In one embodiment, the electronic processor **12** is programmed to determine whether the measured fluid flow through the aspiration catheter **2** exceeds a predetermined fluid flow threshold, and output an alert (e.g., an audio alarm, a video message on the display device **14** of the ultrasound imaging device **10,** and so forth) if the measured fluid flow exceeds the predetermined fluid flow threshold. In some examples, the electronic processor **12** is programmed to automatically turn off or modulate the vacuum suction from the vacuum source **3** if the measured fluid flow exceeds the predetermined fluid flow threshold. In another embodiment, the electronic processor **12** is programmed to detect a decrease in the measured fluid flow through the aspiration catheter as a function of time, and in response to the detected decrease in the measured fluid flow through the aspiration catheter **2,** outputting an indication (e.g., an audio alarm, a video message, and so forth) that the aspiration catheter **2** has engaged the material to be removed (i.e., the vegetation). In some examples, the electronic processor **12** is programmed to automatically set the vacuum suction of the vacuum source **3** to a probing vacuum suction value at an initial stage of the aspiration, and automatically increase the vacuum suction to an aspiration vacuum suction value that is higher than the probing vacuum suction value in response to the detected decrease in the measured fluid flow through the aspiration catheter **2.**

### EXAMPLE

The following describes the aspiration device or apparatus **1** in more detail. The Doppler ultrasound imaging device **10** acquires images with the aspiration catheter **2** in its field of view. When the aspiration is in its off state, the region just beyond the tip of the aspiration catheter **2** does not experience any suction and hence there is no flow in the aspiration catheter **2.** As the aspiration is turned on, blood flows through the aspiration catheter **2.** In 3D imaging mode, the ultrasound b-mode data is captured along with Color Doppler flow information. The b-mode image provides the visualization of aspiration catheter **2** body, its tip, and target vegetation or lesion. The doppler mode provides information of the direction and velocity of blood moving through vessels based on the velocities of the sound scatter. The aspiration catheter **2** appears as a narrow cylinder, the diameter of which is known *a priori.* Flow rate in the blood vessel or the aspiration catheter **2** is defined as the volume of blood passing through the point of measurements per unit time. The Gaussian surface integration of velocity vectors based on the Gauss theorem were proposed with the potential to provide a consistent and accurate method for 3D volumetric blood flow measurements. With the advances in true 3D ultrasound technology, the surface integration method can be easily implemented. Briefly, the method involves obtaining spatial estimates (3D) of velocity vectors using Color flow Doppler ultrasound within the view of the Doppler ultrasound imaging device **10.** These velocity estimates when integrated over a surface intersecting the vessel or catheter would provide the volumetric flow. During aspiration procedure, the feedback can be provided to the user about the flow rate and volume of blood extracted during every aspiration run.

This can allow to provide the operator with warnings or approaching limits as input on the display device **14.** For a lot of patients, the amount of blood that can be removed depends on variety of clinically relevant physiological parameters (e.g., weight, hemoglobin levels). In addition to providing warnings of approaching blood draw limits, patient specific blood draw limits can also be incorporated.

Another challenge during the aspiration procedure is understanding when the aspiration catheter **2** has made contact with the vegetation creating a strong suction. The contact can be verified on imaging alone, but in a dynamic environment where the vegetations can have a 'floating' motion independent of the cardiac and catheter motion. Often, the clinicians will turn the vacuum pump **3** on when making approach to vegetation. This allows them to suck the vegetation into the tip of the aspiration catheter **2.** During this 'fishing' maneuver, there can be quite a bit of loss of blood. In such a scenario, the change in flow rate as calculated by the electronic processor **12** can provide the user with indicator of proper contact. This can be displayed on the display device **14** as a simple binary indication (i.e., contact or no-contact).

In another, as the aspiration catheter **2** approaches the vegetation, there is a change in flow rate through the aspiration catheter **2** due to suction. During this process, calculating the flux (based on known cylindrical inner diameter of the catheter) allows for the monitoring of blood loss and can be used as a control signal to the vacuum pump **3** (motor control; motor current control to vary flow rate for hard lesions, etc.). In such an embodiment, the aspiration catheter **2** can be tethered to the Doppler ultrasound imaging device **10** or the electronic processor **12,** either wired or wirelessly. Based on flow being calculated from the imaging information as well as events based on image features, the aspiration device or apparatus **1** can receive control signals which can modulate the flow rate of the pump. These control signals can be motor current in DC system or be a valve system that can modulate the flow (e.g. pneumatic flow valves).

When the physicians turn on the aspiration as they approach the vegetation, the physicians could greatly benefit with the modulation of aspiration device or apparatus **1.** In such a scenario, based on the imaging feature of the aspiration catheter **2** tip and its proximity to a vegetation, as well as the flow information, the aspiration device or apparatus **1** can be run in a low suction mode. This would allow for a lower extraction volume while helping clinicians make good contact with the vegetation. Once contact is detected on imaging as well as flow signal, the aspiration can be set to a higher suction pressure.

Another scenario that occurs during aspiration is the clogging of the aspiration catheter **2.** Once vegetation accumulates in the aspiration catheter **2,** the aspiration catheter **2** needs to be withdrawn to unclog the aspiration catheter **2.** To help this the vacuum source **3** can be modulated to briefly operate at a higher suction level. This can be done in short pulses of high suction(cyclic suction vs static suction) that can help dislodge the accumulated clot.

In another embodiment, the pattern of flow through the aspiration catheter **2** can be analyzed to detect vegetations being extracted. As the aspiration catheter **2** is filled with blood, the observing vegetations being extracted can be challenging. In addition, the collection of such vegetations in the waste bag needs further observation to confirm extraction. Based on doppler signals, higher density material passing through blood can be identified and detected. This can provide additional level of input to physicians during the aspiration procedure. This information can be presented to the user in the form of a count on the display device **14** for the number/amount of debris passing through the aspiration catheter **2.**

The disclosure has been described with reference to the preferred embodiments. Modifications and alterations may occur to others upon reading and understanding the preceding detailed description. It is intended that the exemplary embodiment be construed as including all such modifications and alterations insofar as they come within the scope of the appended claims or the equivalents thereof.

## Claims

1. A device **(1)** for providing guidance during a procedure to remove tissue vegetation from an implant site using an aspiration catheter **(2)** in which aspiration is controlled by vacuum suction, the device comprising:
a Doppler ultrasound imaging device **(10)** configured to acquire ultrasound data of the implant site including Doppler ultrasound data of the implant site and imaging data of the implant site; and
an electronic processor **(12)** programmed to:
identify a portion of the aspiration catheter in the imaging data of the implant site;
measure fluid flow through the aspiration catheter as a function of time based on the Doppler ultrasound data corresponding to the identified portion of the aspiration catheter; and
the device **characterized in that** the electronic processor further provides guidance for the aspiration of the tissue vegetation based on the measured fluid flow through the aspiration catheter, wherein the guidance includes feedback for controlling the aspiration of the tissue vegetation based on the measured fluid flow.

2. The device **(1)** of claim 1, wherein the Doppler ultrasound imaging device **(10)** is configured to acquire the imaging data of the treatment site as B-mode ultrasound imaging data of the treatment site, and the electronic processor **(12)** is programmed to:
identify the portion of the aspiration catheter in the B-mode imaging data of the treatment site.

3. The device **(1)** of either one of claims 1 and 2, wherein the identifying of the portion of the aspiration catheter **(2)** in the imaging data of the treatment site is based in part on an *a priori* known diameter of the portion of the aspiration catheter.

4. The device **(1)** of any one of claims 1-3, wherein the providing of guidance includes:
determining whether the measured fluid flow through the aspiration catheter **(2)** exceeds a predetermined fluid flow threshold, and
outputting an alert if the measured fluid flow exceeds the predetermined fluid flow threshold.

5. The device **(1)** of claim 4, wherein the electronic processor **(12)** is further programmed to:
automatically turn off or modulate the vacuum suction if the measured fluid flow exceeds the predetermined fluid flow threshold.

6. The device **(1)** of any one of claims 1-5, wherein the providing of guidance includes:
detecting a decrease in the measured fluid flow through the aspiration catheter as a function of time; and
in response to the detected decrease in the measured fluid flow through the aspiration catheter **(2),** outputting an indication that the aspiration catheter has engaged the material to be removed.

7. The device **(1)** of claim 6, wherein the electronic processor **(12)** is further programmed to:
automatically set the vacuum suction to a probing vacuum suction value at an initial stage of the aspiration; and
automatically increase the vacuum suction to an aspiration vacuum suction value that is higher than the probing vacuum suction value in response to the detected decrease in the measured fluid flow through the aspiration catheter **(2).**

8. The device **(1)** of any one of claims 1-7, wherein the electronic processor **(12)** is further programmed to:
measure a density of fluid forming the measured fluid flow based on the Doppler ultrasound data; and
determine an amount of the material to be removed that passes through the aspiration catheter **(2)** based on the measured fluid flow and the measured density.

9. The device **(1)** of any one of claims 1-8, wherein the treatment site is a trans-venous lead anchoring site, the material to be removed is tissue vegetation at the trans-venous lead anchoring site, and the electronic processor **(12)** is further programmed to:
quantify an amount of the tissue vegetation at the trans-venous lead anchoring site based on the imaging data of the treatment site.

10. The device **(1)** of any one of claims 1-9, further including:
an aspiration catheter **(2)** configured to aspirate material to be removed from a treatment site in at least one blood vessel of a patient using vacuum suction on the aspiration catheter.

## Patentansprüche

1. Vorrichtung **(1)** zum Bereitstellen einer Führung während einer Prozedur zum Entfernen von Gewebevegetation an einer Implantationsstelle unter Verwendung eines Aspirationskatheters **(2),** wobei die Aspiration durch Vakuumansaugung gesteuert wird, wobei die Vorrichtung umfasst:
eine Doppler-Ultraschall-Bildgebungsvorrichtung **(10),** die konfiguriert ist, um Ultraschalldaten der Implantationsstelle zu erfassen, die Doppler-Ultraschalldaten der Implantationsstelle und Bildgebungsdaten der Implantationsstelle beinhalten; und
einen elektronischen Prozessor **(12),** der programmiert ist, um:
einen Abschnitt des Aspirationskatheters in den Bildgebungsdaten der Implantationsstelle zu identifizieren;
Fluidfluss durch den Aspirationskatheter in Abhängigkeit von Zeit basierend auf den Doppler-Ultraschalldaten zu messen, die dem identifizierten Abschnitt des Aspirationskatheters entsprechen; und
die Vorrichtung **dadurch gekennzeichnet ist, dass** der elektronische Prozessor weiter Führung für die Aspiration der Gewebevegetation basierend auf dem gemessenen Fluidfluss durch den Aspirationskatheter bereitstellt, wobei die Führung Rückmeldung zur Steuerung der Aspiration der Gewebevegetation basierend auf dem gemessenen Fluidfluss beinhaltet.

2. Vorrichtung (1) nach Anspruch 1, wobei die Doppler-Ultraschall-Bildgebungsvorrichtung (10) konfiguriert ist, um die Bildgebungsdaten der Behandlungsstelle als B-Modus-Ultraschallbildgebungsdaten der Behandlungsstelle zu erfassen, und der elektronische Prozessor **(12)** programmiert ist, um:
den Abschnitt des Aspirationskatheters in den B-Modus-Bildgebungsdaten der Implantationsstelle zu identifizieren.

3. Vorrichtung **(1)** nach einem der Ansprüche 1 und 2, wobei das Identifizieren des Abschnitts des Aspirationskatheters **(2)** in den Bilddaten der Behandlungsstelle teilweise auf einem *a priori* bekannten Durchmesser des Abschnitts des Aspirationskatheters basiert.

4. Vorrichtung **(1)** nach einem der Ansprüche 1-3, wobei das Bereitstellen einer Führung beinhaltet:
Bestimmen, ob der gemessene Fluidfluss durch den Aspirationskatheter **(2)** eine vorbestimmte Flüssigkeitsflussschwelle überschreitet, und
Ausgeben einer Warnung, wenn der gemessene Fluidfluss die vorbestimmte Schwelle überschreitet.

5. Vorrichtung **(1)** nach Anspruch 4, wobei der elektronische Prozessor **(12)** weiter programmiert ist, um:
die Vakuumansaugung automatisch auszuschalten oder zu modulieren, wenn der gemessene Fluidfluss die vorbestimmte Fluidflussschwelle überschreitet.

6. Vorrichtung **(1)** nach einem der Ansprüche 1-5, wobei das Bereitstellen einer Führung beinhaltet:
Erkennen einer Abnahme des gemessenen Fluidflusses durch den Aspirationskatheter in Abhängigkeit von Zeit; und
als Reaktion auf die erkannte Abnahme des gemessenen Fluidflusses durch den Aspirationskatheter **(2)** Ausgeben einer Angabe, dass der Aspirationskatheter das zu entfernende Material in Eingriff genommen hat.

7. Vorrichtung **(1)** nach Anspruch 6, wobei der elektronische Prozessor **(12)** weiter programmiert ist, um:
die Vakuumansaugung automatisch auf einen Sondierungsvakuumansaugwert auf einer Anfangsstufe der Aspiration einzustellen; und
die Vakuumansaugung automatisch auf einen Aspirationsvakuumsaugwert zu erhöhen, der höher ist als der Sondierungsvakuumansaugwert, als Reaktion auf die erkannte Abnahme des gemessenen Fluidflusses durch den Aspirationskatheter **(2).**

8. Vorrichtung **(1)** nach einem der Ansprüche 1-7, wobei der elektronische Prozessor **(12)** weiter programmiert ist, um:
eine Dichte von Fluid, das den gemessenen Fluidfluss bildet, basierend auf den Doppler-Ultraschalldaten zu messen; und
basierend auf dem gemessenen Fluidfluss und der gemessenen Dichte eine Menge des zu entfernenden Materials, die durch den Aspirationskatheter **(2)** verläuft, zu bestimmen.

9. Vorrichtung **(1)** nach einem der Ansprüche 1-8, wobei die Behandlungsstelle eine transvenöse Leitungsverankerungsstelle ist, das zu entfernende Material Gewebevegetation an der transvenösen Leitungsverankerungsstelle ist und der elektronische Prozessor **(12)** weiter programmiert ist, um:
eine Menge der Gewebevegetation an der transvenösen Leitungsverankerungsstelle basierend auf den Bildgebungsdaten der Behandlungsstelle zu quantifizieren.

10. Vorrichtung **(1)** nach einem der Ansprüche 1-9, weiter beinhaltend:
einen Aspirationskatheter **(2),** der konfiguriert ist, um Material, das aus einer Behandlungsstelle in mindestens einem Blutgefäß eines Patienten entfernt werden soll, unter Verwendung von Vakuumansaugung am Aspirationskatheter anzusaugen.

## Revendications

1. Dispositif **(1)** pour fournir un guidage pendant une procédure d'élimination de végétation tissulaire d'un site d'implantation à l'aide d'un cathéter d'aspiration **(2)** dans lequel l'aspiration est commandée par aspiration sous vide, le dispositif comprenant :
un dispositif d'imagerie ultrasonore Doppler **(10)** configuré pour acquérir des données ultrasonores du site d'implantation, y compris des données ultrasonores Doppler du site d'implantation et des données d'imagerie du site d'implantation ; et
un processeur électronique **(12)** programmé pour :
identifier une partie du cathéter d'aspiration dans les données d'imagerie du site d'implantation ;
mesurer le débit de fluide à travers le cathéter d'aspiration en fonction du temps sur la base des données ultrasonores Doppler correspondant à la partie identifiée du cathéter d'aspiration ; et
le dispositif étant **caractérisé en ce que** le processeur électronique fournit en outre un guidage pour l'aspiration de la végétation tissulaire sur la base du débit de fluide mesuré à travers le cathéter d'aspiration, dans lequel le guidage inclut une rétroaction pour commander l'aspiration de la végétation tissulaire sur la base du débit de fluide mesuré.

2. Dispositif (1) selon la revendication 1, dans lequel le dispositif d'imagerie ultrasonore Doppler (10) est configuré pour acquérir les données d'imagerie du site de traitement en tant que données d'imagerie ultrasonore en mode B du site de traitement, et le processeur électronique **(12)** est programmé pour :
identifier la partie du cathéter d'aspiration dans les données d'imagerie en mode B du site de traitement.

3. Dispositif **(1)** selon l'une ou l'autre des revendications 1 et 2, dans lequel l'identification de la partie du cathéter d'aspiration **(2)** dans les données d'imagerie du site de traitement est basée en partie sur un diamètre connu *a priori* de la partie du cathéter d'aspiration.

4. Dispositif **(1)** selon l'une quelconque des revendications 1-3, dans lequel la fourniture d'un guidage inclut :
la détermination établissant si le débit de fluide mesuré à travers le cathéter d'aspiration **(2)** dépasse un seuil de débit de fluide prédéterminé, et
l'émission d'une alerte indiquant si le débit de fluide mesuré dépasse le seuil de débit de fluide prédéterminé.

5. Dispositif **(1)** selon la revendication 4, dans lequel le processeur électronique **(12)** est programmé en outre pour :
désactiver ou moduler automatiquement l'aspiration sous vide si le débit de fluide mesuré dépasse le seuil de débit de fluide prédéterminé.

6. Dispositif **(1)** selon l'une quelconque des revendications 1-5, dans lequel la fourniture d'un guidage inclut :
la détection d'une diminution du débit de fluide mesuré à travers le cathéter d'aspiration en fonction du temps ; et
en réponse à la diminution détectée du débit de fluide mesuré à travers le cathéter d'aspiration **(2),** l'émission d'une indication selon laquelle le cathéter d'aspiration est entré en prise avec la matière à retirer.

7. Dispositif **(1)** selon la revendication 6, dans lequel le processeur électronique **(12)** est programmé en outre pour :
régler automatiquement l'aspiration sous vide à une valeur d'aspiration sous vide de sondage lors d'une étape initiale de l'aspiration ; et
augmenter automatiquement l'aspiration sous vide à une valeur d'aspiration sous vide qui est supérieure à la valeur d'aspiration sous vide de sondage en réponse à la diminution détectée du débit de fluide mesuré à travers le cathéter d'aspiration **(2).**

8. Dispositif **(1)** selon l'une quelconque des revendications 1-7, dans lequel le processeur électronique **(12)** est programmé en outre pour :
mesurer la densité du fluide formant le débit de fluide mesuré sur la base des données ultrasonores Doppler ; et
déterminer une quantité de matière à retirer qui passe à travers le cathéter d'aspiration **(2)** sur la base du débit de fluide mesuré et de la densité mesurée.

9. Dispositif **(1)** selon l'une quelconque des revendications 1-8, dans lequel le site de traitement est un site d'ancrage de sonde transveineuse, la matière à retirer est une végétation tissulaire au niveau du site d'ancrage de sonde transveineuse, et le processeur électronique **(12)** est programmé en outre pour :
quantifier une quantité de végétation tissulaire au niveau du site d'ancrage de sonde transveineuse sur la base des données d'imagerie du site de traitement.

10. Dispositif **(1)** selon l'une quelconque des revendications 1-9, incluant en outre :
un cathéter d'aspiration **(2)** configuré pour aspirer la matière à retirer d'un site de traitement dans au moins un vaisseau sanguin d'un patient à l'aide de l'aspiration sous vide sur le cathéter d'aspiration.
